# EUROPEAN PATENT SPECIFICATION

(11) **EP 2 246 442 B1**
(45) Date of publication and mention of the grant of the patent: **01.08.2018**
(21) Application number: 09173271.9
(22) Date of filing: 16.10.2009
(51) Int. Cl.: C12Q 1/68

(54) **Method for determining sirtuin modulating agents**
Verfahren zur Bestimmung von Sirtuinmodulierungsmitteln
Procédé pour déterminer des agents modulateurs de la sirtuine

(30) Priority: 17.10.2008 BR 08060444
(43) Date of publication of application: 03.11.2010
(73) Proprietor: União Brasileira De Educação E Assistência - Mantenedora Da Pucrs, CEP: 90619-900 Porto Alegre - RS (BR)
(72) Inventor: Reis Bogo, Mauricio, 90630-130 Bairro-Petropolis (BR); Arigony Souto, Andre, 90670-020 Porto Alegre RS (BR); Bonan, Carla Denise, 91350-300 Porto Alegre RS (BR); Carneiro Brandao Pereira, Talita, 90670-020 Porto Alegre RS (BR); Schirmer, Helena, 93700-000 Campo Born RS (BR); Pacheco Rico, Eduardo, 90619-900 Porto Alegre RS (BR); Broock Rosemberg, Denis, 90619-900 Porto Alegre RS (BR)
(74) Representative: Capasso, Olga

(56) References cited:
- WO-A2-2008/115518
- US-A1- 2003 129 724
- US-A1- 2005 171 042
- US-A1- 2007 099 830
- COHEN H Y ET AL: "Calorie restriction promotes mammalian cell survival by inducing the SIRT1 deacetylase" SCIENCE, AMERICAN ASSOCIATION FOR THE ADVANCEMENT OF SCIENCE, WASHINGTON, DC; US LNKD- DOI:10.1126/SCIENCE.1099196, vol. 305, no. 5682, 17 June 2004 (2004-06-17), pages 390-392, XP002379363 ISSN: 0036-8075
- GROZINGER C M ET AL: "Indentification of a Class of Small molecule Inhibitors of the Sirtuin Family of NAD-dependent Deacetylases by Phenotype Screening" JOURNAL OF BIOLOGICAL CHEMISTRY, AMERICAN SOCIETY FOR BIOCHEMISTRY AND MOLECULAR BIOLOGY, INC, US LNKD- DOI:10.1074/JBC.M106779200, vol. 276, no. 42, 1 August 2001 (2001-08-01), pages 38837-38843, XP002392947 ISSN: 0021-9258
- ASHRAF ET AL: "270 POSTER Sirtuin expression correlates with irradiation and paclitaxel treatment in breast cancer" EUROPEAN JOURNAL OF SURGICAL ONCOLOGY, LONDON, GB LNKD- DOI:10.1016/S0748-7983(06)70705-3, vol. 32, 1 November 2006 (2006-11-01), page S81, XP005750323 ISSN: 0748-7983
- BLANDER GIL ET AL: "SIRT1 shows no substrate specificity in vitro" JOURNAL OF BIOLOGICAL CHEMISTRY, AMERICAN SOCIETY FOR BIOCHEMISTRY AND MOLECULAR BIOLOGY, INC, US LNKD- DOI:10.1074/JBC.M414080200, vol. 280, no. 11, 1 March 2005 (2005-03-01), pages 9780-9785, XP002429821 ISSN: 0021-9258
- GARSKE ADAM L ET AL: "SIRT1 top 40 hits: Use of one-bead, one-compound acetyl-peptide libraries and quantum dots to probe deacetylase specificity" BIOCHEMISTRY, AMERICAN CHEMICAL SOCIETY, US LNKD- DOI:10.1021/BI052015L, vol. 45, no. 1, 1 January 2006 (2006-01-01), pages 94-101, XP002429823 ISSN: 0006-2960
- PORCU M ET AL: "The emerging therapeutic potential of sirtuin-interacting drugs: from cell death to lifespan extension" TRENDS IN PHARMACOLOGICAL SCIENCES, ELSEVIER, HAYWARTH, GB LNKD- DOI:10.1016/J.TIPS.2004.12.009, vol. 26, no. 2, 1 February 2005 (2005-02-01), pages 94-103, XP004727629 ISSN: 0165-6147
- GROZINGER C M ET AL: "Deacetylase enzymes: Biological functions and the use of small-molecule inhibitors" CHEMISTRY AND BIOLOGY, CURRENT BIOLOGY, LONDON, GB LNKD- DOI:10.1016/S1074-5521(02)00092-3, vol. 9, no. 1, 1 January 2002 (2002-01-01) , pages 3-16, XP002330125 ISSN: 1074-5521
- HOWITZ K T ET AL: "SMALL MOLECULE ACTIVATORS OF SIRTUINS EXTEND SACCHAROMYCES CEREVISIAE LIFESPAN" NATURE, NATURE PUBLISHING GROUP, LONDON, GB LNKD- DOI:10.1038/NATURE01960, vol. 425, 11 September 2003 (2003-09-11), pages 191-196, XP001188967 ISSN: 0028-0836

## Description

### Field of Invention

The invention herein describes a method for determining sirtuin modulating agents; specifically the modulation of the expression of sirtuins, by way of the detection and comparison of mRNAs of sirtuins and of β-actin.

### Invention Background

Sirtuins are part of a large group of enzymes, the histone deacetylases or HDACs family, whose principal role is to reverse the regulatory acetylation of histone-type proteins, by influencing directly in the structure of nucleosomes and, consequently, in gene transcription. Other than histones, a growing number of proteins have been identified as a target of HDACs, structural proteins and different transcription factors being among those. Sirtuins affect a broad number of physiological processes, including regulation of life expectancy, regulation of metabolic and enzymatic activity, cellular response to stress, neurodegeneration, DNA repair, rDNA recombination, apoptosis and the control of cellular proliferation. Currently, sirtuins are the important targets of research linked to caloric restriction, cancer, neurodegenerative diseases, muscular differentiation, inflammation and obesity.

Different studies have been performed aiding in the identification of sirtuin modulators. Blander et al ("SIRT1 shows no substrate specificity in vitro" J Biol Chem. 280(1 1):9780-5, 2005) describe a previously unpublished method for identifying specificity for a sequence of deacetylase by using peptide libraries containing acetylated lysine. After incubation with SIRT1, the subassembly of deacetylated peptides was captured selectively using a biotinylated N-hydroxysuccinimide photolabile linker and small beads of streptavidin, subsequently analysed. Said studies revealed that the recognition of the substrate by SIRT1 occurs irrespectively of the sequence of amino acids close to the acetylated lysine. Furthermore, Garske et al. ("SIRTI top 40 hits: use of one-bead, one-compound peptide libraries and quantum dots to probe deacetylase specificity." Biochemistry 45(I):94-101, 2006) also describe an new method of high scalability for determining the specificity of deacetylase substrates using an acetylated peptide library of one-bead, one-compound (OBOC). A library of 104,907 unique peptides was constructed and screened utilising NAD+- dependent deacetylase SIRT1 for more efficient peptide sequences. As a result, it was discovered that SIRT1 can discriminate between peptide substrates depending on the context in which they find themselves.

The document, WO 08/27379, describes a method for monitoring the modulation of sirtuin activity by determining the level of acetylation of sirtuin substrates.

Documents, WO 07/149270 and WO 06/096780, describe a method for screening modulating molecules of sirtuin activity, which include as a crucial stage the determination of the level of acetylation of a specific peptide.

The document, WO 07/084162, describes new molecules with sirtuin inhibiting activity that are useful in the treatment and/or prevention of cancer and autoimmune diseases.

The document US 2003/129724 relates to genes encoding histone deacetylase class II proteins.

The document WO 2008/115518 describes methods for monitoring sirtuin modulation and methods for identifying compounds that modulate the activity of a sirtuin protein.

The document US 2007/0099830 is concerned with the ADP-ribosyl transferase activity of Sirt4.

The document US 2005/0171042 describes compounds, compositions and methods for modulating the expression of Histone deacetylase 1.

The abovementioned studies illustrate the difficulties and discrepancies that arise from methods for identifying specific substrates for sirtuins. With regards peptide libraries, such libraries were limited to small sequences and are influenced by amino acids that can be incorporated and they provide information about artificial sequences in an artificial context.

As the methods proposed by publicly disclosed research do not demonstrate a consensus regarding their results, one can see that there exists a need for a method for identifying sirtuin modulators and in an appropriate cellular environment. The identification of modulating agents will help to clarify the role of sirtuins in cells and will aid in the development of diagnostic tests and the treatment of patients with modulators of the respective activity, as well as methods for identifying new modulating compounds.

The invention herein is different from all the documents relating to publicly disclosed research, as it proposes a new method for determining the modulation of sirtuin activity through the modulation of its gene expression. None of the documents from publicly disclosed research describes or even suggests that sirtuin activity can be modulated with agents that modulates its expression. Until now, publicly disclosed research has been concerned solely with the modulation of enzymatic sirtuin activity, which has already been expressed in the cell.

### Description of the Invention

Firstly, the invention herein provides a method for determining modulating agents of sirtuins in tissue cell(s) comprising the following stages:
a. contacting tissue cell(s) comprising at least one gene of the sirtuin family and the β-actin gene, with a modulating agent;
b. determining the relative abundance of mRNA of the sirtuin-family gene;
c. determining the relative abundance of the mRNA of the β-actin gene; and
d. determining the mRNA SIRT/mRNA β-actin ratio;
wherein if the ratio is greater than 1, the modulating agent is a positive modulator and if the ratio is lower than 1, the modulating agent is a negative modulator and wherein the tissue cell is selected from the group consisting of cells of fish, birds, and mammals.

Preferably, the cells are cells from Danio rerio. Gallus gallus, Mus musculus or Homo Sapiens. Preferably, the tissue is chosen from the group consisting of brain, kidney, sexual organs (male and female), spleen, gills, liver, muscle, heart and combinations of the same.

In a preferred embodiment, the sirtuin possesses at least 50% of identity with at least one sequence chosen from the group consisting of XP40_001334440, AAH67165, XP_684225, XP_690925, AAH83418, AAH75987, NP_001002071, XP_698800, Q96EB6, Q8IXJ6, Q9NTG7, Q9Y6E7, Q9NXA8, Q8N6T7, Q9NRC8, BAD38898, BAS38897, XP_420920, XP_415273, XP_418925, NP_001034409, NP_001026277, NP_062786, NP_071877, CAJ18608, Q8R216, NP_849179, NP_853617, AAP83960 and combinations of the same.

In another preferred embodiment, the β-actin possess at least 50% of identity with the NM_001101 sequence.

Preferably, the relative abundance of mRNA of sirtuins and/or β-actin is determined by PCR.

### Description of the Figures

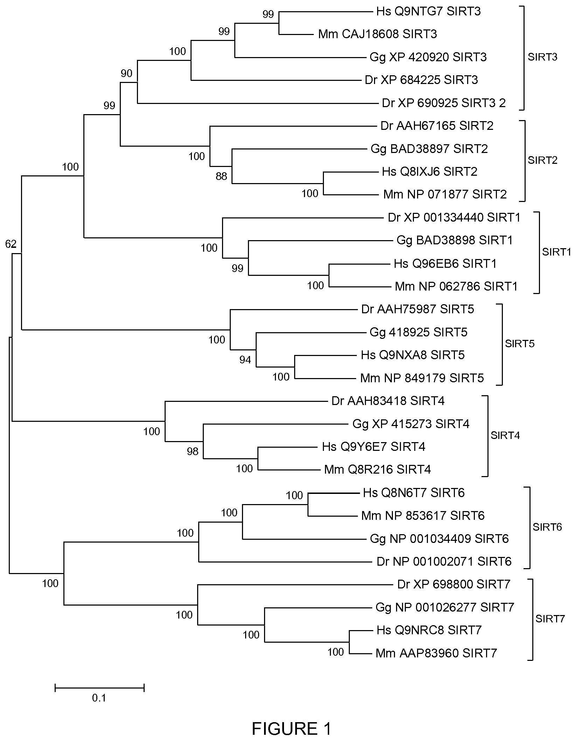

Figure 1 demonstrates one of the trees generated that was constructed utilising a proportional distance (p-distance) by way of the Neighbour-Joining (NJ) method, using the MEGA4.02 program. The tree contains seven well-settled terminal cladistic groupings with a high support amount, corresponding to each one of the sirtuins described. The phylogenetic tree consistently grouped together the orthological sequences, SIRT1-SIRT7 of *Danio rerio* (Dr), *Gallus gallus* (Gg), *Mus musculus* (Mm) and *Homo sapiens* (Hs)

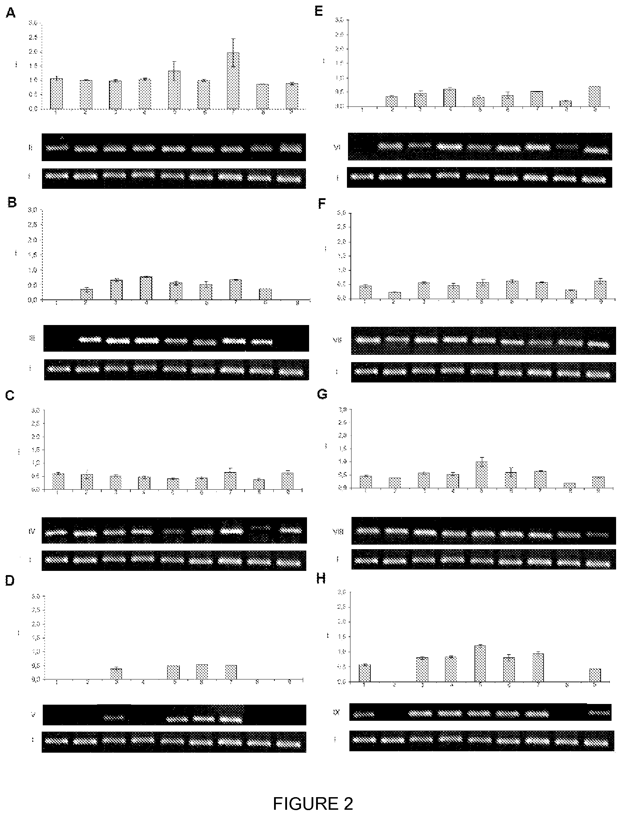

Figure 2 demonstrates the pattern of expression of SIRT1 (A), SIRT2 (B), SIRT3 (C), SIRT3.2 (D), SIRT4 (E), SIRT5 (F), SIRT6 (G) and SIRT7 (H) in *zebrafish.* These results are expressed as an mRNA ratio of sirtuin/ β-actin (mean ± S.E.) from seven repeated, independent experiments. On the Y axis, I is the rate of mRNA, on the X axis, 1 is the spleen, 2 is the gills, 3 is the brain, 4 is the heart, 5 is the liver, 6 is the female sexual organ, 7 is the male sexual organ, 8 is muscle and 9 is the kidney, I is β-actin, II is SIRT1, III is SIRT2, IV is SIRT3, V is SIRT3.2, VI is SIRT4, VII is SIRT5, VIII is SIRT6, IX is SIRT7.

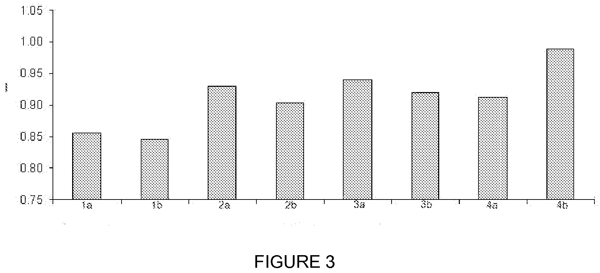

Figure 3 demonstrates the evaluation of the effect of resveratrol in the modulation of sirtuin activity in zebrafish in different tissues in 4 animals (groups 1 to 4 on the X axis). On the Y axis, I is the rate of mRNA X, a is muscle and b is liver.

### Detailed Description of the Invention

The examples shown here have the single aim of exemplifying one of the numerous ways of carrying out the invention, however, without detracting from its purpose.

### Sirtuins

For the purpose of this invention, the expression, "sirtuins", refers to the sequences (DNA and/or RNA), as well as the respective proteins, comprising at least 50% of identity with the sequences (DNA and/or RNA), as well as the respective proteins of the chosen genes of the group comprising SIRT1, SIRT2, SIRT3, SIRT4, SIRT5, SIRT5, SIRT6, SIRT7 and combinations of the same.

The respective sequences of the SIRT1, SIRT2, SIRT3, SIRT4, SIRT5, SIRT6 and SIRT7 genes are described in the GenBank, under the access codes for the species, *Danio rerio* (XP_001334440, AAH67165, XP_684225, XP_690925 (SIRT 3.2), AAH83418, AAH75987, NP_001002071, XP_698800), *Homo sapiens* (Q96EB6, Q8IXJ6, Q9NTG7, Q9Y6E7, Q9NXA8, Q8N6T7, Q9NRC8), *Mus musculus* (BAD38898, BAS38897, XP_420920, XP_415273, XP_418925, NP_001034409, NP_001026277) and *Gallus gallus* (NP_062786, NP_071877, CAJ18608, Q8R216, NP_849179, NP_853617, AAP83960).

Ideally, sirtuins that are useful in the invention herein possess DNA sequences with at least 50% identity with the sequences illustrated in Figure 1.

### β-Actin

For the purposes of this invention, the expression, "β-actin" refers to sequences (DNA and/or RNA), as well as respective proteins, comprising at least 50% identity with the sequences (DNA and/or RNA), as well as respective proteins of the β-actin gene.

Ideally, the β-actin sequence is described in the GenBank under the access number, NM_001101.

Cells comprising at least of gene of the sirtuin family

For the purposes of this invention, adequate cells are cells of vertebrate animals. The vertebrate should be chosen from the group consisting of fish, birds, mammals and combinations of the same.

Ideally, the vertebrate should be chosen from the group comprising *Danio rerio, Gallus gallus, Mus musculus, Homo sapiens* and combinations of the same.

The cell is chosen from different tissues in the animal. Examples of organs/tissues include, but are not limited to, the brain, kidney, sexual organs (male and female), spleen, gills, liver, muscle, heart and combinations of the same.

The *zebrafish* (*Danio rerio*) possess numerous advantageous characteristics that firstly make it the principal vertebrate model for biology studies in development and genetics and, subsequently, expansion to other areas of biological sciences. The *zebrafish* combines the characteristic of being a vertebrate with the study size of an invertebrate organism. The knowledge accumulated from the *"Zebrafish* Genome Project" together with the capability to quickly absorb chemical substances directly from water have the effect that it is increasingly used as well as a model species for studies in toxicology, pharmacology and human diseases.

The identification of genes related to sirtuins in *zebrafish* genomes and the determination of expression patterns in different tissues constitute a very powerful tool for screening modulating molecules for the activities of these proteins.

### Method for determining Modulating Agents

The method for determining modulating agents of sirtuin gene expression is comprised of the following stages:
a) contacting cells comprising at least one gene of the sirtuin family and the β-actin gene, with a modulating agent;
b) determining the relative abundance of mRNA of the sirtuin-family gene;
c) determining the relative abundance of the mRNA of the β-actin gene; and
d) determining the mRNA *SIRTI*mRNA *β-actin* ratio.

Ideally, one determines the relative abundance by way of mRNA amplification tests in both genes. Said tests are common knowledge to experts in the subject and do not need to be described here in greater detail.

In the event that the compound possesses a mRNA *SIRTI*mRNA *β-actin* ratio that is greater than 1, the compound is classified as a positive modulator (amplifier) of sirtuin gene expression.

In the event that the compound possesses a mRNA *SIRTI*mRNA *β-actin* ratio that is less than 1, the compound is classified as a negative modulator (inhibitor) of sirtuin gene expression.

### Pharmaceutical composition

For the purposes of this invention, pharmaceutical compositions include all compositions that contain an active principle, with prophylactic, palliative and/or curative purposes, which acts so as to maintain and/or restore homeostasis. It can be administered topically, parenterally, enterally and/or intrathecally.

The expression, "pharmaceutically acceptable", is employed here when referring to compounds, materials, compositions and/or dosage methods which, within the medical field, are appropriate for use in contact with human and animal tissue without excessive toxicity, irritation, allergic response or other problems or complications, it having a reasonable benefit/risk relationship.

The composition in the invention herein can be administered by way of an oral dosage, in the form of tablets, capsules (each one includes sustained liberation or formulations with liberation time), pills, powders, granules, elixirs, dyes, suspensions, syrups and emulsions.

The pharmaceutical composition in this invention is comprised of:
a) a modulating agent for sirtuin expression; and
b) a vehicle which is acceptable pharmaceutically,
in which the mRNA *SIRTI*mRNA *β-actin* ratio of the cell without the presence of the modulating agent is different from the mRNA *SIRTI*mRNA *β-actin* ratio of the cell without the presence of the modulating agent.

### Example

Eight genes related to sirtuins were identified in the *zebrafish* genome *SIRT1-7* and a *SIRT3* paralogue, named *SIRT3.2* (Table 1) utilising the sequences deduced from *Homo sapiens* amino acids (Q96EB6, Q8IXJ6, Q9NTG7, Q9Y6E7, Q9NXA8, Q8N6T7, Q9NRC8) *Mus musculus* (BAD38898, BAD38897, Xp_420920, XP_415273, XP_418925, NP_001034409, NP_001026277) and *Gallus gallus* (NP_062786, NP_071877, CAJ18608, Q8R216, NP_849179, NP_853617, AAP83960). The identity of each one of the eight sequences of *zebrafish* was confirmed by way of phylogenetic analysis (Figure 1)

**Table 1. Sequences Summary: Access number, primers sequence and PCR amplification conditions.**

| | | PCR Conditions | | |
|---|---|---|---|---|
| Sirtuin | GenBank ID | Primers (5' - 3') | Tm (°C) | Ciclos |
| SIRT 1 | XP_001334440 | F - CAGCTCTGCTACAATTCATCGCGTC | 62 | 30 |
| | | R - AATCTCTGTAGAGTCCAGCGCGTGTG | | |
| SIRT 2 | AAH67165.1 | F - TCTCTGAAGAAATTCCTAAGTGCGATTCC | 61 | 30 |
| | | R - TTATCTGAATCAAAATCCATTCCGCCTC | | |
| SIRT 3 | NP_001073643 | F - CATTAAATGTGGTGGAACAAGAGGCCTG | 61 | 30 |
| | | R - AGTTCCTCTCCTTTGTAATCCCTCCGAC | | |
| SIRT 3.2 | NP_001038173.1 | F - CGGCAGGCTGATGAAGCTTGGTCG | 63 | 30 |
| | | R - TAGCTTGCTTGGCTTCCTCTGCAGG | | |
| SIRT 4 | AAH83418.1 | F - TGTGGTGAACTGACTCCTCGTGCTGAGC | 63 | 30 |
| | | R - CGGAAGTTTTCTTTCACTAGCAGCGAGG | | |
| SIRT 5 | AAH75987.1 | F - CCACGGTAGTCTGTTTAAAACCCGCTG | 61 | 30 |
| | | R - AGTGATATTTGAAGCGTTGGGTAGCAGG | | |
| SIRT 6 | NP_001002071 | F - GGACTGGGAGGACTCTCTGCCCGAC | 68 | 30 |
| | | R - GCCCGGCCCACTCCGGAACG | | |
| SIRT 7 | AAI55852.1 | F - GCATTTTGGAGAACGTGGCACTTTGG | 61 | 45 |
| | | R - GTTTAGCCATGCTGAAGATGGGGTCC | | |

The mapping of the pattern and levels of expression in each one of the eight genes related to *zebrafish* sirtuin was determined in eight tissues (spleen, gills, brain, heart, liver, female sexual organs and male sexual organs, skeletal muscle and kidneys), by way of the analysis of semi-quantitative RT-PCR comparing the relative abundance of mRNA of each one of the genes related to sirtuins with the mRNA of the gene codifying for β-actin (mean ± E.P.) (Figure 2 and Table 2).

**Table 2. Gene expression pattern: relative expression of mRNA in genes related to sirtuin in different zebrafish tissues.**

| Tissues | Optical Density (O.D.) for *SIRTIβ-actin* mRNA ratio (mean ± S.E) | | | | | | | |
|---|---|---|---|---|---|---|---|---|
| | *SIRT1* | *SIRT2* | *SIRT3* | *SIRT3.2* | *SIRT4* | *SIRT5* | *SIRT6* | *SIRT7* |
| Spleen | 1.07±0.07 | * | 0.61±0.05 | * | * | 0.44±0.05 | 0.47±0.03^{g} | 0.56±0.04^{d,g} |
| Gills | 1.01±0.01 | 0.34±0.00^{a,d,e} | 0.57±0.16 | * | 0.36±0.10^{f} | 0.24±0.01^{c,f} | 0.38±0.01^{g} | * |
| Brain | 0.98±0.05 | 0.65±0.08^{b} | 0.52±0.04 | 0.37±0.03 | 0.46±0.03 | 0.55±0.03 | 0.56±0.04^{g,h} | 0.79±0.10^{g} |
| Heart | 1.05±0.04 | 0.77±0.05^{b,h} | 0.46±0.05 | * | 0.61±0.07^{h} | 0.45±0.09 | 0.53±0.06^{g,h} | 0.83±0.05 |
| Liver | 1.33±0.34 | 0.56±0.02 | 0.41±0.04 | 0.49±0.09 | 0.33±0.06^{f} | 0.56±0.12 | 0.98±0.17^{a,b,c.d,e,f,h} | 1.19±0.04^{a,c,f,i} |
| Female Sexual Organ | 1.00±0.04 | 0.51±0.06 | 0.43±0.03 | 0.54±0.07 | 0.39±0.06 | 0.61±0.05^{b} | 0.59±0.17^{g,h} | 0.79±0.05^{g} |
| Male Sexual Organ | 1.97±0.49^{h,i} | 0.68±0.09^{b} | 0.64±0.17 | 0.51±0.08 | 0.52±0.12^{h} | 0.56±0.01 | 0.65±0.03^{g,h} | 0.92±0.11^{f,i} |
| Skeletal Muscle | 0.87±0.01^{g} | 0.37±0.01^{e} | 0.38±0.05 | * | 0.19±0.01^{e,d,f} | 0.29±0.02^{f} | 0.19±0.00^{a,c.d,e,f} | * |
| Kidney | 0.89±0.04^{g} | * | 0.64±0.06 | * | 0.69±0.03^{b,g,m} | 0.62±0.09^{b,h} | 0.41±0.02^{g} | 0.45±0.09^{d,g} |

| | | | | | | | | |
|---|---|---|---|---|---|---|---|---|
| The results were analysed by ANOVA followed by the post-hoc Tukey HSD test, considering P≤0.05 as significant. The mRNA levels were significantly different from ^{a}spleen, ^{b}gills, ^{c}brain, ^{d}heart, ^{e}liver, ^{f}female sexual organ, ^{g}male masculine organ, ^{h}skeletal muscle, ⁱkidney. | | | | | | | | |

So as to evaluate modulation in this protein family, the effect of resveratrol (3.4', 5-trihydroxy-trans-stilbene), a phytoalexin which is found is some food items, such as egg shell, peanuts and red wine, was evaluated and it promoted a significant increase in *SIRT1* gene expression in skeletal muscle and liver in the proposed model (Figure 3).

## Claims

1. Method for determining modulating agents of sirtuins in tissue cell(s) comprising the following stages:
a. Contacting tissue cell(s) comprising at least one gene of the sirtuin family and the β-actin gene, with a modulating agent;
b. determining the relative abundance of mRNA of the sirtuin-family gene;
c. determining the relative abundance of the mRNA of the β-actin gene; and
d. determining the mRNA SIRT/mRNA β-actin ratio;
wherein if the ratio is greater than 1, the modulating agent is a positive modulator and if the ratio is lower than 1, the modulating agent is a negative modulator and wherein the tissue cell is selected from the group consisting of cells of fish, birds, and mammals.

2. Method for determination, pursuant to claim 1, in which the cells are cells from *Danio rerio, Gallus gallus, Mus musculus* or *Homo Sapiens.*

3. Method for determination, pursuant to claims 1 and 2, in which the tissue is chosen from the group consisting of brain, kidney, sexual organs (male and female), spleen, gills, liver, muscle, heart and combinations of the same.

4. Method for determination, pursuant to claim 1, in which the sirtuin possesses at least 50% of identity with at least one sequence chosen from the group consisting of XP_001334440, AAH67165, XP_684225, XP_690925, AAH83418, AAH75987, NP_001002071, XP_698800, Q96EB6, Q8IXJ6, Q9NTG7, Q9Y6E7, Q9NXA8, Q8N6T7, Q9NRC8, BAD38898, BAS38897, XP_420920, XP_415273, XP_418925, NP_001034409, NP_001026277, NP_062786, NP_071877, CAJ18608, Q8R216, NP_849179, NP_853617, AAP83960 and combinations of the same.

5. Method for determination, pursuant to claim 1, in which the β-actin possess at least 50% of identity with the NM_001101 sequence.

6. Method for determination, pursuant to claim 1, in which the relative abundance of mRNA of sirtuins and/or β-actin is determined by PCR.

## Patentansprüche

1. Verfahren zur Bestimmung von Modulationsmitteln von Sirtuinen in einer Gewebezelle/in Gewebezellen, umfassend die folgenden Schritte:
a. Inkontaktbringen von einer Gewebezelle/von Gewebezellen, die mindestens ein Gen der Sirtuin-Familie und das β-Aktin-Gen umfassen, mit einem Modulationsmittel;
b. Bestimmen der relativen Häufigkeit der mRNA des Gens von der Sirtuin-Familie;
c. Bestimmen der relativen Häufigkeit der mRNA des Gens von β-Aktin; und
d. Bestimmen des Verhältnisses mRNA SIRT/mRNA β-Aktin;
wobei, wenn das Verhältnis größer als 1 ist, das Modulationsmittel ein positiver Modulator ist und wenn das Verhältnis kleiner als 1 ist, das Modulationsmittel ein negativer Modulator ist und wobei die Gewebezelle ausgewählt ist aus der Gruppe, bestehend aus Zellen von Fischen, Vögeln und Säugetieren.

2. Verfahren zur Bestimmung nach Anspruch 1, bei dem die Zellen die Zellen von *Danio rerio, Gallus gallus, Mus musculus* oder *Homo Sapiens* sind.

3. Verfahren zur Bestimmung nach den Ansprüchen 1 und 2, bei dem das Gewebe aus der Gruppe, bestehend aus Gehirn, Niere, Geschlechtsorganen (männlich und weiblich), Milz, Kiemen, Leber, Muskel, Herz und Kombinationen derselben ausgewählt wird.

4. Verfahren zur Bestimmung nach Anspruch 1, bei dem das Sirtuin mindestens 50 % Identität mit mindestens einer Sequenz besitzt, die aus der Gruppe, bestehend aus XP_001334440, AAH67165, XP_684225, XP_690925, AAH83418, AAH75987, NP_001002071, XP_698800, Q96EB6, Q8IXJ6, Q9NTG7, Q9Y6E7, Q9NXA8, Q8N6T7, Q9NRC8, BAD38898, BAS38897, XP_420920, XP_415273, XP_418925, NP_001034409, NP_001026277, NP_062786, NP_071877, CAJ18608, Q8R216, NP_849179, NP_853617, AAP83960 und Kombinationen derselben ausgewählt ist.

5. Verfahren zur Bestimmung nach Anspruch 1, bei dem das β-Aktin mindestens 50 % der Identität mit der NM_001101-Sequenz besitzt.

6. Verfahren zur Bestimmung nach Anspruch 1, bei dem die relative Häufigkeit von mRNA von Sirtuinen und/oder β-Aktin durch PCR bestimmt wird.

## Revendications

1. Procédé de détermination d'agents modulateurs de sirtuines dans une(des) cellule(s) de tissu comprenant les étapes suivantes :
a. mise en contact d'une(de) cellule(s) de tissu comprenant au moins un gène de la famille des sirtuines et le gène de la β-actine, avec un agent modulateur ;
b. détermination de l'abondance relative de l'ARNm du gène de la famille des sirtuines ;
c. détermination de l'abondance relative de l'ARNm du gène de la β-actine ; et
d. détermination du rapport d'ARNm de SIRT/ARNm de β-actine ;
où si le rapport est plus grand que 1, l'agent modulateur est un modulateur positif et si le rapport est inférieur à 1, l'agent modulateur est un modulateur négatif et où la cellule de tissu est choisie dans le groupe constitué par les cellules de poisson, d'oiseaux et de mammifères.

2. Procédé de détermination, selon la revendication 1, dans lequel le cellules sont des cellules de *Danio rerio, Gallus gallus, Mus musculus* ou *Homo sapiens.*

3. Procédé de détermination, selon les revendications 1 et 2, dans lequel le tissu est choisi dans le groupe constitué par le cerveau, le rein, les organes sexuels (mâle et femelle), la rate, les ouïes, le foie, le muscle, le coeur et leurs combinaisons.

4. Procédé de détermination, selon la revendication 1, dans lequel la sirtuine possède au moins 50 % d'identité avec au moins une séquence choisie dans le groupe constitué par XP_001334440, AAH67165, XP_684225, XP_690925, AAH83418, AAH75987, NP_001002071, XP_698800, Q96EB6, Q8IXJ6, Q9NTG7, Q9Y6E7, Q9NXA8, Q8N6T7, Q9NRC8, BAD38898, BAS38897, XP_420920, XP_415273, XP_418925, NP_001034409, NP_001026277, NP_062786 ; NP_071877, CAJ18608, Q8R216, NP_849179, NP_853617, AAP83960 et leurs combinaisons.

5. Procédé de détermination, selon la revendication 1, dans lequel la β-actine possède au moins 50 % d'identité avec la séquence NM _001101.

6. Procédé de détermination, selon la revendication 1, dans lequel l'abondance relative d'ARNm de sirtuines et/ou de β-actine est déterminée par PCR.
